# EUROPÄISCHE PATENTSCHRIFT

(11) **EP 2 233 469 B1**
(45) Veröffentlichungstag und Bekanntmachung des Hinweises auf die Patenterteilung: **18.07.2012**
(21) Anmeldenummer: 09155232.3
(22) Anmeldetag: 16.03.2009
(51) Int. Cl.: C07D 207/08, C07D 231/12

(54) **Verfahren zur Kopplung von Halogen-substituierten Aromaten mit Trialkylsilyl-substituierten Heteroatom-haltigen organischen Verbindungen**
Method for coupling halogen-substituted aromatic substances with trialkylsilyl-substituted heteroatom-containing organic compounds
Procédé de couplage d'aromates substitués par des halogènes, dotés de liaisons organiques contenant des atomes hétéroclytes substitués par du trialkylsilyl

(43) Veröffentlichungstag der Anmeldung: 29.09.2010
(73) Patentinhaber: Zylum Beteiligungsgesellschaft mbH & Co. Patente II KG, 12529 Schönefeld / Waltersdorf (DE)
(72) Erfinder: Thiel, Prof. Dr. Werner R., 676661 Kaiserslautern (DE); Böge, Dr. Nicolas, 20149 Hamburg (DE); Kreipl, Dr. Andreas, 20359 Hamburg (DE)
(74) Vertreter: Zech, Stefan Markus

(56) Entgegenhaltungen:
- US-A1- 2008 042 127
- M. MURATA ET AL: "Palladium-catalyszed cross-coupling reaction of aryltriethosysilanes with aryl bromides under basic aqueous conditions" SYNTHESIS, Bd. 15, 2001, Seiten 2231-2233, XP002530801 Stuttgart
- "Aldrich Katalog Feinchemikalien und Laborgeräte 2003/2004" 2003, ALDRICH , XP002530803 Seite 1159, ganze Seite; Seite 1665, 1. Verbindung
- "Aldrich Katalog Feinchemikalien und Laborgeräte 2003/2004" 2003, , XP002530804 ganze Seiten 1322 -1325
- C. H. BURGOS ET AL: "Significantly improved method for the Pd-catalyzed coupling of phenols with aryl halide: understanding ligand effects" ANGEWANDTE CHEMIE, Bd. 45, 2006, Seiten 4321-4326, XP002530802 Weinheim

## Beschreibung

Die Erfindung betrifft ein Verfahren zur Herstellung von Aryl-Heteroatom-verbrückten Verbindungen, bei denen ein Aryl-Rest unmittelbar an ein Heteroatom, insbesondere N oder O, gebunden ist, das seinerseits Teil eines Heteroatom-haltigen organischen Rests ist.

Aryl-Heteroatom-verbrückte Verbindungen sind wertvolle Ausgangssubstanzen für die organische Synthese. Sie stellen insbesondere wertvolle Vorläuferverbindungen für die Synthese von pharmazeutischen Wirkstoffen sowie von Pestiziden und Herbiziden dar.

Die bisherigen Synthesen solcher Verbindung basieren auf einer kombinatorischen Strategie und erfordern eine geeignete Kupplungsmethode mit teilweise komplexer Schutzgruppenstrategie oder mit Übergangsmetall-katalysierten Schritten. Der Einsatz von Schutzgruppen ist mit dem Nachteil verbunden, dass der Einbau der Schutzgruppe vor der Kupplung und die anschließende Abspaltung der Schutzgruppe mit zusätzlichen Reaktionsschritten verbunden sind, was die Komplexität der Verfahrensführung deutlich steigert und somit zu höheren Kosten und größerer Störanfälligkeit des Synthesewegs führen. Darüber hinaus muss eine Schutzgruppenstrategie auf die jeweils zu schützende funktionelle Gruppe sowie die weiteren im Reaktionsansatz vorhandenen Substanzen abgestimmt werden, so dass sich kein universell einsetzbares Verfahrensschema ergibt, das für verschiedenste Umsetzungen dieses Typs einsetzbar wäre.

Der Einsatz von Übergangsmetallkatalysatoren ist in der organischen Synthese weit verbreitet. Solche Übergangsmetallkatalysatoren sind jedoch mit dem Nachteil verbunden, dass sie einerseits oftmals recht teuer sind und andererseits das Produkt der Umsetzung mit toxischen Schwermetallverbindungen kontaminieren können. Gerade bei Verwendung der Substanzen als Ausgangssubstanz für die Synthese pharmazeutischer Wirkstoffe ist dies ein schwerwiegender Nachteil, der aufwendige und kostenintensive Reinigungsschritte notwendig macht.

So handelt es sich beispielsweise bei der Buchwald-Hartwig Kreuz-Kupplungsreaktion um eine Palladium-katalysierte Synthese vom Arylamin, die nach folgenden Reaktionsschema abläuft:

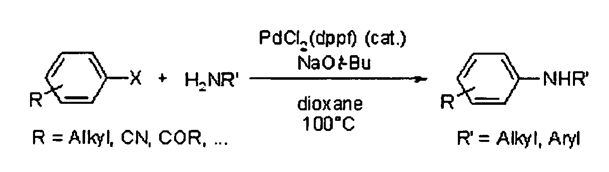

Aus der jüngeren Literatur bekannte Beispiele für Reaktionen dieses Typs sind:

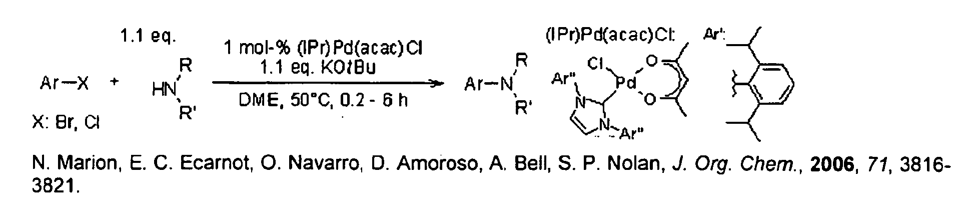

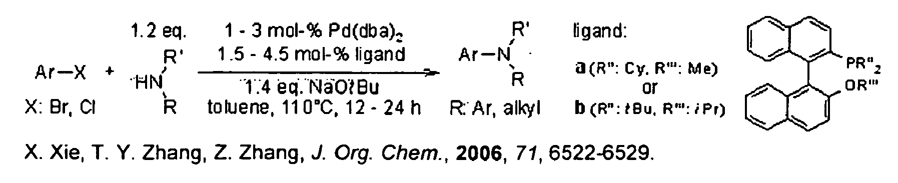

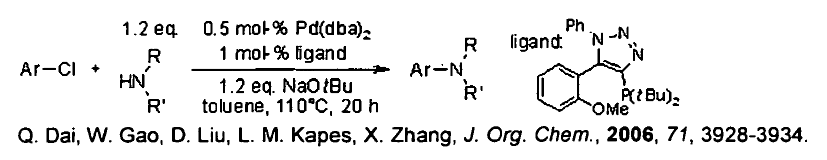

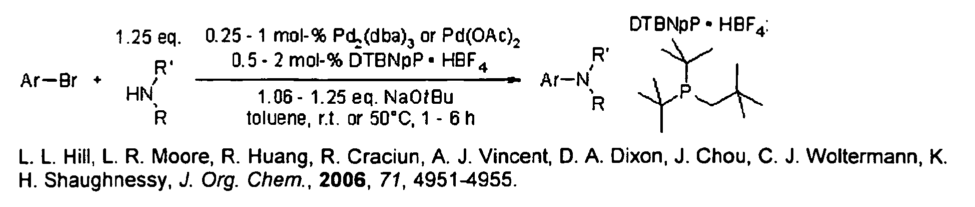

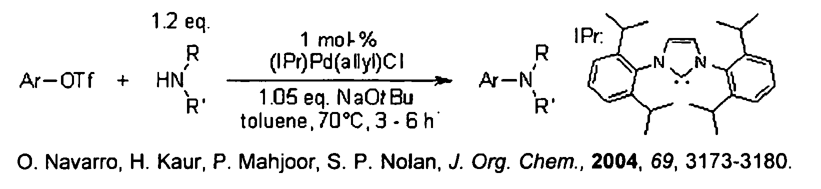

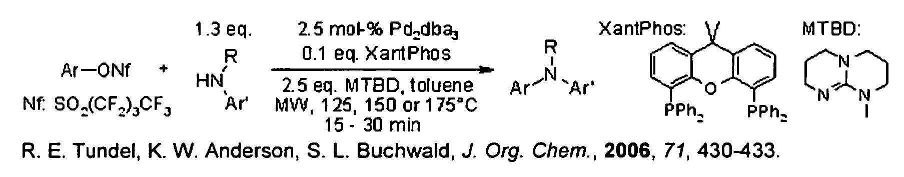

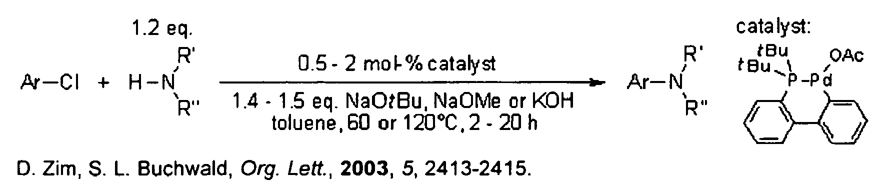

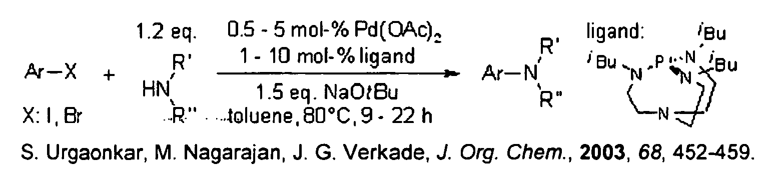

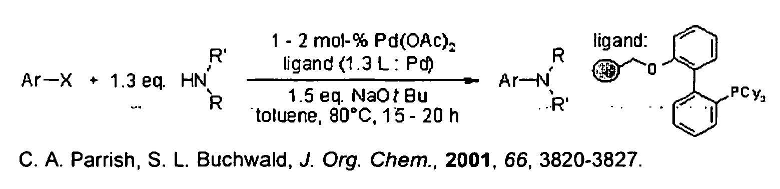

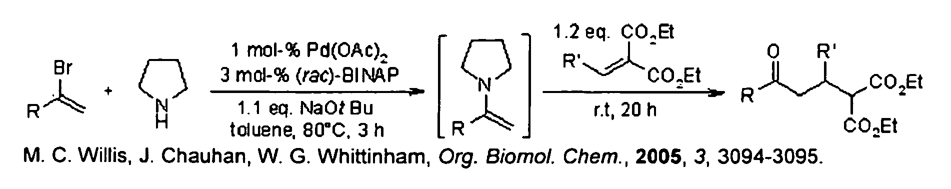

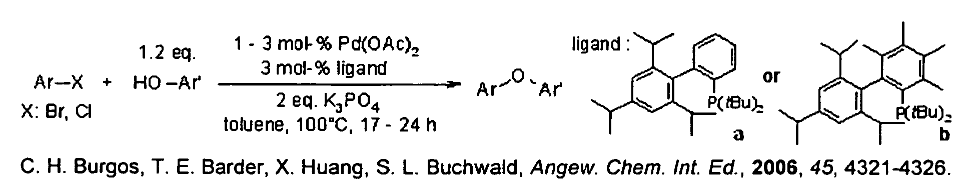

Insgesamt ist festzustellen, dass die bisherigen Verfahren zur Herstellung von Aryl-Heteroatom-verbrückten Verbindungen durch Kreuzkupplung unter Einsatz von Schutzgruppen eine aufwendige Verfahrensführung bedingen und somit zu hohen Kosten führen. Darüber hinaus werden mit diesen Methoden meist relativ geringe Ausbeuten erhalten. Die Übergangsmetall-katalysierten Reaktionen sind mit dem Nachteil der Kontamination des Endproduktes mit toxischen Schwermetallen verbunden.

Die Verwendung von Trialkylsilylgruppierungen als Substituenten in der organischen Synthese hat in den letzten Jahren eine gewisse Bedeutung erlangt. Typischerweise werden Trialkylsilylgruppen dabei als Schutzgruppen eingesetzt, die verhindern sollen, dass im Laufe einer mehrstufigen Synthese vorhandene funktionelle Gruppen mit eingesetzten Reagenzien reagieren. Es wurden aber auch einige Synthesen beschrieben, in denen Trialkylsilylsubstituenten unmittelbar an der Umsetzung beteiligt sind.

Ein Beispiel für eine Reaktion dieses Typs ist die Hiyama Kopplung, bei der eine Palladium-katalysierte C-C Bindungsbildung zwischen Aryl, Alkenyl oder Alkyl Halogeniden oder Pseudohalogeniden und Organosilanen nach folgenden Reaktionsschema stattfindet:

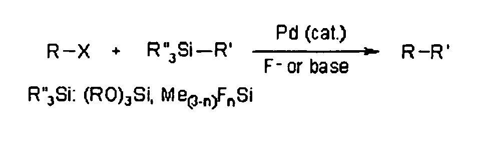

Beispiele aus der jüngeren Literatur für Reaktionen dieses Typs sind:

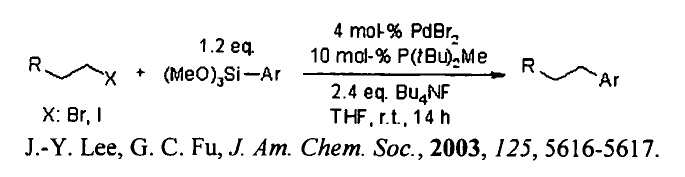

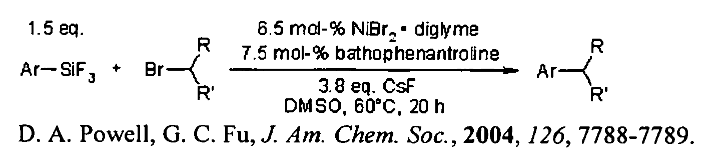

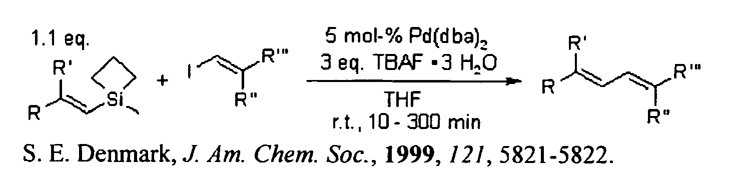

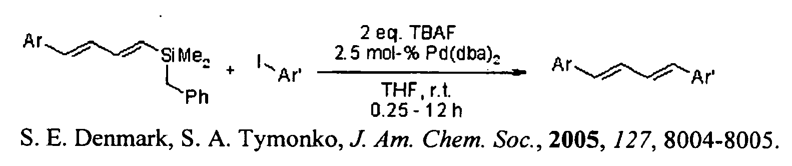

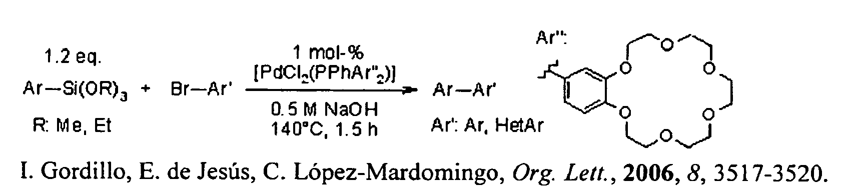

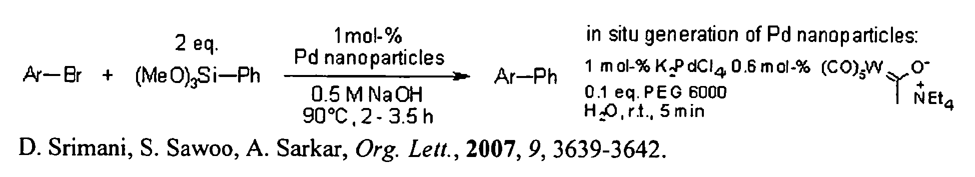

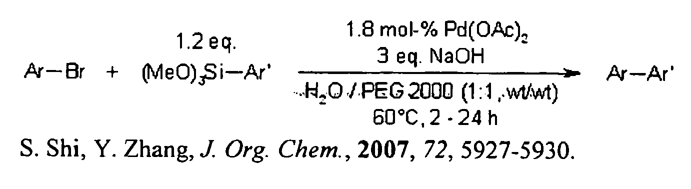

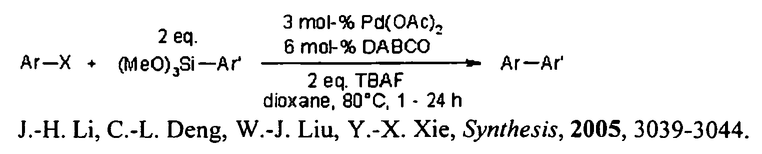

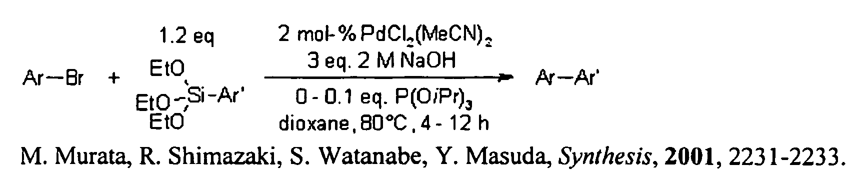

Die DE 37 38 276 beschreibt ein Verfahren zur Herstellung von Polyarylensulfiden, bei dem die Polyarylensulfide durch Schmelzkondensation aus Halogenarylthiosilanen hergestellt werden. Die Umsetzung findet in Gegenwart mindestens eines Alkali- und/oder Erdalkali- bzw. Ammoniumhalogenids als Katalysator statt.

Die US 2008-0042127 beschreibt die Kupplung von verbrückten Silylverbindungen mit perfluorierten Aromaten unter Verwendung von Fluorid-Ionen zur Darstellung von Verbindungen, welche zur Produktion von elektronischen Bauteilen geeignet sind.

Die US 5523384 beschreibt die Darstellung von Polyetherketonen unter Einsatz von Silanolaten und Kupfer-Katalysatoren.

M. Murata et al.: "Palladium-catalyszed cross-coupling reaction of aryltriethosysilanes with aryl bromides under basic aqueous conditions" Synthesis, Bd. 15, 2001, Seiten 2231-2233, Stuttgart beschreibt das Reaktionsschema einer Kreuzkupplung zwischen einem Halogen-substituierten Aromat und einer Silyl-substituierten heteroatom-organischen Verbindung. Bei der siliziumhaltigen Abgangsgruppe gemäß diesem Reaktionsschema handelt es sich jedoch nicht um eine Ethoxylgruppe. Im Übrigen wird in dieser Publikation die Verwendung eines edelmetallhaltigen Katalysators als essentiell für die Reaktion dargestellt.

US 2008/042127 beschreibt Reaktionen zur Kopplung von substituierten Aromaten. Die Kopplung eines Halogen-substituierten Aromaten mit einer Trialkylsilyl-substituierten Heteroatom-haltigen organischen Verbindung aus einer Stoffklasse der Phosphane, Pyramidine, Pyrazole, Pyrole, Oxazole, Pyrrolidine, Imidazole und/oder Triazole wird in dieser Publikation nicht beschrieben.

C.H. Burgos et al.: "Significally improved method for the PD-catalyzed coupling of Phenols with aryl halide: understanding ligand effects", Angewandte Chemie, Bd. 45, 2006, Seiten 4321-4326, Weinheim, beschreibt ein Verfahren zur Kopplung von Phenolen mit Arylhaliden. Ein Verfahren zur Herstellung von Aryl-Heteroatom-verknüpften Verbindungen wird in dieser Publikation nicht beschrieben.

Wie oben beschrieben, leiden alle bisher bekannten Verfahren zur Herstellung von Aryl-Heteroatom-verbrückten Verbindungen unter deutlichen Nachteilen, insbesondere im Hinblick auf Komplexität der Reaktionsführung, die erzielbaren Ausbeuten und auf die unter Umständen vorhandenen toxischen Kontaminationen im Endprodukt.

Angesichts dessen lag der vorliegenden Erfindung die Aufgabe zugrunde, ein Verfahren zur Herstellung von Aryl-Heteroatom-verbrückten Verbindungen, bei denen ein Aryl-Rest unmittelbar an ein Heteroatom gebunden ist, das seinerseits Teil eines Heteroatom-haltigen organischen Restes ist, bereitzustellen, das gegenüber den vorhergehenden Verfahren einfacher, schneller und kostengünstiger durchzuführen ist, zu hohen Ausbeuten führt, und die Kontamination der Endprodukte mit toxischen Schwermetallen vermeidet.

Die vorstehend genannte Aufgabe wird durch ein Verfahren gelöst, das dadurch gekennzeichnet ist, dass ein Halogen-substituierter Aromat mit einer Trialkylsilyl-substituierten Heteroatom-haltigen organischen Verbindung umgesetzt wird, wobei es sich bei der Trialkylsilyl-substituierten Heteroatom-haltigen organischen Verbindung um eine Verbindung aus einer der Stoffklassen der Phosphane, Pyrimidine, Pyrazole, Pyrole, Oxazole, Pyrolidine, Imidazole und/oder Triazole handelt.

Es hat sich überraschend gezeigt, dass durch diese Synthesestrategie die vorher schwer zugänglichen Aryl-Heteroatom-verbrückten Verbindungen in einer Ein-Schritt Synthese mit hohen Ausbeuten erhalten werden können. Die Reaktion läuft unter milden Bedingungen ohne den Einsatz Übergangsmetall-haltiger Katalysatoren ab.

In einer bevorzugen Ausführungsform wird als Halogen-substituierter Aromat ein Mono- oder Di-Halogen-substituierter Aromat eingesetzt.

Besonders gute Ergebnisse werden erzielt, wenn als Halogen-substituierter Aromat ein Fluor-substituierter Aromat eingesetzt wird.

Des Weiteren wird in einer bevorzugten Ausführungsform als Trialkylsilyl-substituierte Heteroatom-haltige organische Verbindung eine Trimethylsilyl-substituierte Heteroatom-haltige organische Verbindung eingesetzt.

Das Verfahren eignet sich grundsätzlich zur Darstellung von Aryl-Heteroatom-verbrückten Verbindungen verschiedenster Strukturen. Durch den Einsatz der leicht zugänglich Trialkylsilyl-aktivierten Heteroatom-haltigen organischen Verbindungen wird eine große Vielzahl unterschiedlichster Aryl-Heteroatom-verbrückter Verbindungen in einem Reaktionsschritt unter hohen Ausbeuten zugänglich. Insbesondere handelt es sich bei der Trialkylsilylsubstituierten Heteroatom-haltigen Verbindung um eine Verbindung, bei der die Trialkylsilylgruppe(n) als Abgangsgruppe(n) an das /die zu verbrückende(n) Heteroatom(e) gebunden ist/sind.

In einer besonders bevorzugten Ausführungsform handelt es sich bei der Trialkylsilyl-substituierten Heteroatom-haltigen organischen Verbindung um eine Verbindung aus einer der Stoffklassen der Phosphane, Pyrimidine, Pyrazole, Pyrole, Oxazole, Pyrolidine, Imidazole und/oder Triazole.

In einer alternativen erfindungsgemäßen Ausführungsform handelt es sich bei der Trialkylsilylsubstituierten Heteroatom-haltigen organischen Verbindung um eine azyklische Verbindung, die eine Kupplung über O oder N erlaubt.

Einer der wichtigsten Vorteile des erfindungsgemäßen Verfahrens besteht darin, dass auf den Einsatz Übergangsmetall-haltiger Katalysatoren verzichtet werden kann. Allerdings werden besonders gute Ergebnisse bei Einsatz von Substanzen aus der Gruppe der Halogenide der Alkali- oder Erdalkalimetalle oder eines Ammoniumhalogenids erzielt. Diesen Substanzen wirken als Katalysatoren der beschriebenen Reaktion, so dass in der Regel nur geringe Mengen eingesetzt werden. Typischerweise werden diese Substanzen in einer Menge von 0,1 bis 100 Mol%, insbesondere in einer Menge von 5 bis 50 Mol%, bezogen auf den Halogen-substituierten Aromaten eingesetzt. In einer bevorzugten Ausführungsform findet die Umsetzung somit in Gegenwart einer zumindest katalytischen Menge eines Halogenids der Alkali- oder Erdalkalimetalle oder eines Ammoniumhalogenids statt.

Es hat sich gezeigt, dass besonders gute Ergebnisse erzielt werden, wenn die Umsetzung in Gegenwart einer zumindest katalytischen Menge von Caesiumfluorid stattfindet. In einer besonders bevorzugten Ausführungsform wird das Caesiumfluorid in einer Menge von 0,1 bis 100 Mol%, insbesondere in einer Menge von 5 bis 50 Mol%, bezogen auf den Halogen-substituierten Aromaten, eingesetzt.

Ein weiterer wichtiger Vorzug des erfindungsgemäßen Verfahrens besteht darin, dass unter besonders milden Bedingungen gearbeitet werden kann. Insbesondere werden keine hohen Temperaturen benötigt, um nach relativ kurzen Reaktionszeiten hohe Ausbeuten zu erhalten. In einer bevorzugten Ausführungsform findet die Umsetzung bei einer Temperatur im Bereich von 0 bis 120°C, insbesondere von 40 bis 90°C statt. Oftmals kann sogar bei Raumtemperatur gearbeitet werden, was die Verfahrensführung weiter vereinfacht.

Als Lösungsmittel für die Umsetzung können verschiedenste in der organischen Synthese verwendbare Lösungsmittel eingesetzt werden. Besonders gute Ergebnisse werden erzielt, wenn die Umsetzung in einem Lösungsmittel, ausgewählt aus der Gruppe, die aus DMF (Dimethylformamid), DMSO (Dimethylsulfoxyd), N-Methylpyrolidin und THF (Tetrahydrofuran) besteht, stattfindet.

Die Erfindung betrifft auch die Aryl-Heteroatom-verbrückten Verbindungen, nach die nach dem vorstehend beschriebenen Verfahren hergestellt sind.

Die so hergestellten Aryl-Heteroatom-verbrückten Verbindungen stellen wertvolle Ausgangssubstanzen für die organische Synthese dar. Besondere Bedeutung haben sie als Ausgangssubstanzen für die Synthese von Wirkstoffen verschiedenster Arten. Die Erfindung betrifft somit auch die Verwendung der nach dem erfindungsgemäßen Verfahren hergestellten Aryl-Heteroatom-verbrückten Verbindung zur Herstellung von Verbindungen aus der Gruppe der pharmazeutischen Verbindungen, Pestizide und/oder Herbizide.

Zusammenfassend ist festzustellen, dass das es das erfindungsgemäße Verfahren erlaubt, Aryl-Heteroatom-verbrückte Verbindungen in einfacher, schneller und kostengünstiger Weise unter milden Bedingungen zu synthetisieren und dabei den Einsatz von Übergangsmetallhaltigen Katalysatoren zu vermeiden.

Die oben beschriebene Erfindung wird im Folgenden anhand von Ausführungsbeispielen noch näher erläutert:

### Beispiel 1:

### Synthese von 4-Imidazol-1-ylbenzonitril

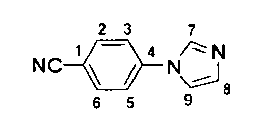

Unter Stickstoffatmosphäre wurden 362 mg (2.38 mmol) CsF, das vorher mit NaOH aktiviert wurde, in 5 ml DMF suspendiert und für 30 min gerührt. Anschließend wurde 1.00 g (8.26 mmol) 4-Fluorbenzonitril zugesetzt. Nach 10 min wurden 1.20 ml (8.18 mmol) N-Trimethylsilylimidazol zugegeben und die Mischung für 20 h bei 60°C gerührt. Zur Aufarbeitung wurde das Lösungsmittel im Ölpumpenvakuum weitgehend entfernt und dann der Reaktionsansatz mit 5 ml Wasser und 5 ml CH2Cl2 versetzt. Die organische Phase wurde abgetrennt, die wässrige Phase mit CH2Cl2 extrahiert, die organischen Phasen vereinigt, mehrfach mit Wasser gewaschen und mit MgS04 getrocknet. Das Lösungsmittel wurde im Vakuum entfernt, und der verbleibende Feststoff zweimal mit Pentan gewaschen.
Ausbeute: 1.07 g (6.31 mmol, 77 %), Aussehen: farbloser Feststoff. 1H-NMR (CDCl3, 25°C, 400.13 MHz): δ = 7.25 (s, 1H, ImH), 7.33 (s, 1H, ImH), 7.51-7.53 (m, 2H, PhH), 7.79-7.81 (m, 2H, PhH), 7.94 (s, 1H, ImH). 13C-NMR (CDCl3, 25 °C, 100.61 MHz): δ = 111.3 (C-1), 117.7 (C-9), 117.9 (-CN), 121.5 (C-3, C-5), 131.6 (C-8), 134.2 (C-2, C-6), 135.4 (C-7) 140.6 (C-4).

### Beispiel 2:

### Synthese von 4-Pyrrolidin-1-ylbenzonitril

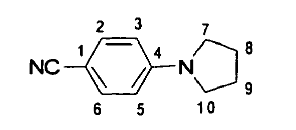

Unter Stickstoffatmosphäre wurden 320 mg (2.11 mmol) CsF in 5 ml DMF suspendiert und für 30 min gerührt. Anschließend wurde 1.00 g (8.26 mmol) 4-Fluorbenzonitril zugesetzt. Nach 10 min wurden 1.44 ml (8.25 mmol) N-Trimethylsilylpyrrolidin zugegeben und die Mischung für 6 d bei Raumtemperatur gerührt. Zur Aufarbeitung wurde der Reaktionsansatz mit 5 ml Wasser und 5 ml CH2Cl2 versetzt. Die organische Phase wurde abgetrennt, die wässrige Phase mit CH2Cl2 extrahiert, die organischen Phasen vereinigt, mehrfach mit Wasser gewaschen und mit MgS04 getrocknet und das Lösungsmittel im Vakuum entfernt.
Ausbeute: 1.01 g (5.87 mmol, 71 %), Aussehen: hellgelber Feststoff. 1H-NMR (CDCl3, 25 °C, 400.13 MHz): δ = 2.00-2.07 (m, 4H, H-8, H-9), 3.30-3.34 (m, 4H, H-7, H-10), 6.47-6.51 (m, 2H, H-3, H-5), 7.42-7.46 (m, 2H, H-2, H-6). 13C-NMR (CDCl3, 25 °C, 100.61 MHz): δ = 25.5 (C-8, C-9), 47.6 (C-7, C-10), 96.7 (C-1), 111.6 (C-3, C-5), 121.1 (-CN), 133.6 (C-2, C-6), 150.1 (C-4).

### Beispiel 3:

### Synthese von 4-(3,5-Dimethylpyrazol-1-yl)benzonitril

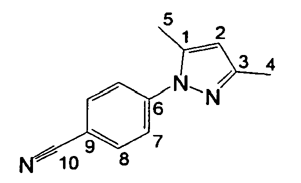

Zu 0.36 g (2.14 mmol) 1-Trimethylsilyl-3,5-dimethylpyrazol wurden 5.0 ml trockenes DMF, 0.26 g (2.15 mmol) 4-Fluorbenzonitril und 0.07 g (0.46 mmol) CsF gegeben. Das gelbe Reaktionsgemisch färbt sich im Folgenden grün. Nach 20 h werden 25 ml CH2Cl2 und 20 ml Wasser zugegeben und die wässrige Phase dreimal mit je 10 ml CH2Cl2 extrahiert. Die vereinigten organischen Auszüge werden dreimal mit je 25 ml Wasser gewaschen, mit MgS04 getrocknet und am Rotationsverdampfer von leichtflüchtigen Bestandteilen befreit.
Ausbeute: 0.26 g (1.32 mmol, 62 %). C12H11N3 - 1H-NMR (CDCl3, 200.13 MHz): □ = 2.11 (s, 3 H, H-5), 2.23 (d, 3 H, H-4), 5.89 (s, 1 H, H-2), 7.46 (d, 3J = 8.6 Hz, 2 H, H-7), 7.58 (d, 3J = 8.5 Hz, 2 H, H-8) ppm. - 13C-NMR (CDCl3, 50.32 MHz): □ = 12.5 (s, 1 C, C-4), 13.0 (s, 1 C, C-5), 108.5 (s, 1 C, C-2), 109.5 (s, 1 C, C-9), 117.9 (s, 1 C, C-10), 123.5 (s, 2 C, C-7), 132.7 (s, 2 C, C-8), 139.3 (s, 1 C, C-1), 143.1 (s, 1 C, C-6), 150.0 (s, 1 C, C-3) ppm.

### Beispiel 4:

### Synthese von 1-(4-Nitrophenyl)pyrrolidin

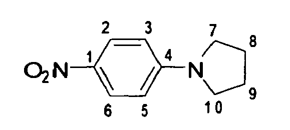

Unter Stickstoffatmosphäre wurden 340 mg (2.24 mmol) Caesiumfluorid, das vorher mit Natriumhydroxid aktiviert wurde, in 5 ml DMF suspendiert und für 30 min gerührt. Anschließend wurden 1.14 g (8.08 mmol) 4-Fluornitrobenzol zugesetzt. Nach 10 min wurden 1.44 ml (8.25 mmol) N-Trimethylsilylpyrrolidin zugegeben und die Mischung für 6 h bei 60 °C gerührt. Zur Aufarbeitung wurde das DMF im Ölpumpenvakuum weitgehend entfernt und dann der Reaktionsansatz mit 15 ml Wasser und 20 ml Dichlormethan versetzt. Die organische Phase wurde abgetrennt, die wässrige Phase mit Dichlormethan extrahiert, die organischen Phasen vereinigt, mehrfach mit Wasser gewaschen und mit Magnesiumsulfat getrocknet und das Lösungsmittel im Vakuum entfernt.

Ausbeute: 1.37 g (7.14 mmol, 88 %), Aussehen: orangefarbener, kristalliner Feststoff. 1H-NMR (CDCl3, 25 °C, 400.13 MHz): δ = 2.07-2.11 (m, 4H, H-8, H-9), 3.40-3.44 (m, 4H, H-7, H-10), 6.47-6.50 (m, 2H, H-3, H-5), 8.12-8.15 (m, 2H, H-2, H-6). 13C-NMR (CDCl3, 25 °C, 100.61 MHz): δ = 25.5 (C-8, C-9), 48.0 (C-7, C-10), 110.5 (C-3, C-5), 126.5 (C-2, C-6), 136.6 (C-1), 152.0 (C-4).

### Beispiel 5:

### Synthese von 4-Pyrrolidin-1-yl-benzoesäuremethylester

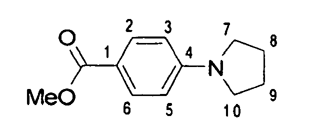

Unter Stickstoffatmosphäre wurden 368 mg (2.42 mmol) Caesiumfluorid, das vorher mit Natriumhydroxid aktiviert wurde, in 5 ml DMF suspendiert und für 30 min gerührt. Anschließend wurden 1.25 g (8.11 mmol) 4-Fluorbenzoesäuremethylester zugesetzt. Nach 10 min wurden 1.44 ml (8.25 mmol) N-Trimethylsilylpyrrolidin zugegeben und die Mischung für 110 h bei 60 °C gerührt. Zur Aufarbeitung wurde das DMF im Ölpumpenvakuum weitgehend entfernt und dann der Reaktionsansatz mit 15 ml Wasser und 25 ml Dichlormethan versetzt. Die organische Phase wurde abgetrennt, die wässrige Phase mit Dichlormethan extrahiert, die organischen Phasen vereinigt, mehrfach mit Wasser gewaschen und mit Magnesiumsulfat getrocknet und das Lösungsmittel im Vakuum entfernt.

Ausbeute: 1.19 g (5.80 mmol, 72 %), Aussehen: farbloser kristalliner Feststoff. 1H-NMR (CDCl3, 25 °C, 400.13 MHz): δ = 2.00-2.03 (m, 4H, H-8, H-9), 3.32-3.35 (m, 4H, H-7, H-10), 6.48-6.51 (m, 2H, H-3, H-5), 7.88-7.90 (m, 2H, H-2, H-6). 13C-NMR (CDCl3, 25 °C, 100.62 MHz): δ = 25.6 (C-8, C-9), 47.7 (C-7, C-10), 51.4 (CH3), 110.9 (C-3, C-5), 116.8 (C-1), 131.5 (C-2, C-6), 151.1 (C-4), 167.7 (C=O).

## Patentansprüche

1. Verfahren zur Herstellung von Aryl-Heteroatom-verbrückten Verbindungen, bei denen ein Aryl-Rest unmittelbar an ein Heteroatom gebunden ist, das seinerseits Teil eines Heteroatom-haltigen organischen Restes ist, **dadurch gekennzeichnet, dass** ein Halogen-substituierter Aromat mit einer Trialkylsilylsubstituierten Heteroatom-haltigen organischen Verbindung umgesetzt wird, wobei es sich bei der Trialkylsilyl-substituierten Heteroatom-haltigen organischen Verbindung um eine Verbindung aus einer der Stoffklassen der Phosphane, Pyrimidine, Pyrazole, Pyrole, Oxazole, Pyrolidine, Imidazole und/oder Triazole handelt.

2. Verfahren nach Anspruch 1, **dadurch gekennzeichnet, dass** als Halogen-substituierter Aromat ein Mono- oder Di-Halogen substituierter Aromat eingesetzt wird.

3. Verfahren nach Anspruch 1 oder 2, **dadurch gekennzeichnet, dass** als Halogen-substituierter Aromat ein Fluor-substituierter Aromat eingesetzt wird.

4. Verfahren nach mindestens einem der vorhergehenden Ansprüche, **dadurch gekennzeichnet, dass** als Trialkylsilyl-substituierte Heteroatom-haltige organische Verbindung eine Trimethylsilyl-substituierte Heteroatom-haltige organische Verbindung eingesetzt wird.

5. Verfahren nach mindestens einem der vorhergehenden Ansprüche, **dadurch gekennzeichnet, dass** die Umsetzung in Gegenwart einer zumindest katalytischen Menge eines Halogenids der Alkali- oder Erdalkalimetalle oder eines Ammoniumhalogenids stattfindet.

6. Verfahren nach Anspruch 5, **dadurch gekennzeichnet, dass** die Umsetzung in Gegenwart einer zumindest katalytischen Menge von Caesiumfluorid stattfindet.

7. Verfahren nach mindestens einem der vorhergehenden Ansprüche, **dadurch gekennzeichnet, dass** die Umsetzung bei einer Temperatur im Bereich von 0 bis 120°C, insbesondere 40 bis 90°C stattfindet.

8. Verfahren nach mindestens einem der vorhergehenden Ansprüche, **dadurch gekennzeichnet, dass** die Umsetzung in einem Lösungsmittel, ausgewählt aus der Gruppe, die aus DMF, DMSO, N-Methylpyrolidin und THF besteht, stattfindet.

## Claims

1. A method for preparing aryl heteroatom-bridged compounds in which an aryl radical is bound directly to a heteroatom which in turn is part of a heteroatom-containing organic radical, **characterized in that** a halogen substituted aromatic compound is reacted with a trialkylsilyl substituted heteroatom-containing organic compound, wherein the trialkylsilyl substituted heteroatom-containing organic compound is a compound from one of the substance classes of phosphanes, pyrimidines, pyrazoles, pyroles, oxazoles, pyrrolidines, imidazoles and/or triazoles.

2. The method according to claim 1, **characterized in that** a monohalogen or dihalogen substituted aromatic compound is employed as the halogen substituted aromatic compound.

3. The method according to claim 1 or 2, **characterized in that** a fluorine substituted aromatic compound is employed as the halogen substituted aromatic coumpound.

4. The method according to at least one of the preceding claims, **characterized in that** a trimethylsilyl substituted heteroatom-containing organic compound is employed as the trialkylsilyl substituted heteroatom-containing organic compound.

5. The method according to at least one of the preceding claims, **characterized in that** the conversion takes place in the presence of an at least catalytic amount of a halide of alkali metals or alkaline earth metals or an ammonium halide.

6. The method according to claim 5, **characterized in that** the conversion takes place in the presence of an at least catalytic amount of caesium fluoride.

7. The method according to at least one of the preceding claims, **characterized in that** the conversion takes place at a temperature ranging from 0 to 120°C, e.g. 40 to 90°C.

8. The method according to at least one of the preceding claims, **characterized in that** the conversion takes place in a solvent selected from the group consisting of DMF, DMSO, N-methylpyrrolidine and THF.

## Revendications

1. Procédé de production de liaisons pontées par un hétéroatome avec un aryle, dans lesquelles un reste d'aryle est directement lié à un hétéroatome, lequel fait partie d'un reste organique renfermant un hétéroatome, **caractérisé en ce qu'**un composé aromatique halogéno-substitué est transformé avec une liaison organique renfermant un hétéroatome et substituée par un trialkylsilyle, la liaison organique renfermant un hétéroatome et substituée par un trialkylsilyle étant une liaison provenant de l'une des classes de matières suivantes : phosphanes, pyrimidines, pyrazoles, pyroles, oxazoles, pyrrolidines, imidazoles et/ou triazoles.

2. Procédé selon la revendication 1, **caractérisé en ce qu'**un composé aromatique substitué par un monohalogène ou un dihalogène est utilisé comme composé aromatique halogéno-substitué.

3. Procédé selon la revendication 1 ou 2, **caractérisé en ce qu'**un composé aromatique substitué par un fluor est utilisé comme composé aromatique halogéno-substitué.

4. Procédé selon au moins l'une des revendications précédentes, **caractérisé en ce qu'**une liaison organique renfermant un hétéroatome et substituée par un triméthylsilyle est utilisée comme liaison organique renfermant un hétéroatome et substituée par un trialkylsilyle.

5. Procédé selon au moins l'une des revendications précédentes, **caractérisé en ce que** la transformation a lieu en présence d'une quantité au moins catalytique d'un halogénide des métaux alcalins ou alcalino-terreux ou d'un halogénide d'ammonium.

6. Procédé selon la revendication 5, **caractérisé en ce que** la transformation a lieu en présence d'une quantité au moins catalytique de fluorure de césium.

7. Procédé selon au moins l'une des revendications précédentes, **caractérisé en ce que** la transformation a lieu à une température comprise dans une plage de 0 à 120 °C, par exemple de 40 à 90 °C.

8. Procédé selon au moins l'une des revendications précédentes, **caractérisé en ce que** la transformation a lieu dans un solvant sélectionné dans le groupe comprenant DMF, DMSO, N-méthylpyrrolidine et THF.
